# EUROPEAN PATENT APPLICATION

(11) **EP 4 810 628 A1**
(43) Date of publication of application: **23.09.2026**
(21) Application number: 24897435.4
(22) Date of filing: 21.11.2024
(51) Int. Cl.: C12Q 1/689

(54) **METHOD AND APPARATUS FOR EVALUATING RISK OF ALZHEIMER-TYPE DEMENTIA**

(30) Priority: 29.11.2023 WO PCT/JP2023/042753
(71) Applicant: Ohara, Tadashi, Sendai-shi, Miyagi 980-8575 (JP)
(72) Inventor: Ohara, Tadashi, Sendai-shi, Miyagi 980-8575 (JP)
(74) Representative: Isarpatent
(86) International application number: PCT/JP2024/041270
(87) International publication number: WO 2025/115749

(57) **Abstract**

The method of assessing the risk of Alzheimer's dementia according to the present invention includes the steps of: extracting DNA from a test sample; identifying bacterial species contained in the test sample based on the sequence data of the extracted DNA; outputting the respective abundances of bacterial species belonging to a first group and bacterial species belonging to a second group, or the relative abundance ratio of the bacterial species belonging to the first group and the bacterial species belonging to the second group, of the bacteria contained in the test sample; and assessing the abundances or the relative abundance ratio. The test sample is a fecal sample. The first group includes bacterial species associated with Alzheimer's dementia. The second group includes bacterial species associated with the inhibition of Alzheimer's dementia.

## Description

### Technical Field

The present disclosure relates to a method and an apparatus for assessing the risk of Alzheimer's dementia. This application claims priority to PCT application No. PCT/JP2023/42753, filed on November 29, 2023, the entire disclosure of which is incorporated herein by reference.

### Background Art

Efforts have been made to assess health status or the risk of diseases based on gut microbiota. Patent Literature 1 discloses a method of classifying the type of gut microbiota for assessing the risk of a disease, as such a technique. The type classification method disclosed in Patent Literature 1 includes a measurement step of measuring the relative abundances of nucleic acids derived from specific bacteria in a test sample, and a classification step of classifying the type of gut microbiota in the test sample based on the relative abundances and a predetermined reference value. Patent Literature 1 discloses 36 specific names of genera, as the specific bacteria.

Patent Literature 2 discloses a method of diagnosing the probability of a subject developing dementia, or the probability of the subject having dementia, based on the abundances of specific bacteria contained in a gut microbiome sample of the subject. The diagnosis method disclosed in Patent Literature 2 includes comparing the ratio of *Firmicutes* and *Bacteroidetes* contained in the gut microbiome sample, in particular.

### Citation List

### Patent Literature

Patent Literature 1: JP 7193810 B
Patent Literature 2: JP 2023-508968 A

### Summary of Invention

### Technical Problem

The relationship between gut microbiota and dementia has been examined, for example, as described in Patent Literature 2. Now that dementia is becoming a significant social issue, however, there are ongoing needs for novel methods of assessing the risk of dementia.

In view of such circumstances, an object of the present disclosure is to provide a novel method and apparatus for assessing the risk of Alzheimer's dementia.

### Solution to Problem

The method of assessing the risk of Alzheimer's dementia according to the present disclosure includes the steps of:
extracting DNA contained in a test sample from the test sample;
identifying bacterial species contained in the test sample based on the sequence data of the extracted DNA;
outputting the abundances of bacterial species belonging to a first group and bacterial species belonging to a second group, or the relative abundance ratio of the bacterial species belonging to the first group and the bacterial species belonging to the second group, of the bacterial species contained in the test sample; and
assessing the abundances or the relative abundance ratio.
The test sample is a fecal sample. The first group includes bacterial species associated with Alzheimer's dementia. The second group includes bacterial species associated with the inhibition of Alzheimer's dementia.

The apparatus for assessing the risk of Alzheimer's dementia according to the present disclosure includes:
a microbiome data acquisition unit that extracts DNA contained in a test sample from the test sample, and identifies bacterial species contained in the test sample based on the sequence data of the extracted DNA;
a data derivation unit that outputs the abundances of bacterial species belonging to a first group and bacterial species belonging to a second group, or the relative abundance ratio of the bacterial species belonging to the first group and the bacterial species belonging to the second group, of the bacterial species contained in the test sample, based on the microbiome data of the test sample, acquired by the microbiome data acquisition unit; and
an assessment unit that assesses the risk of Alzheimer's dementia based on the result derived by the data derivation unit.
The test sample is a fecal sample. The first group includes bacterial species associated with Alzheimer's dementia. The second group includes bacterial species associated with the inhibition of Alzheimer's dementia.

### Advantageous Effects of Invention

The present disclosure provides a novel method and apparatus for assessing the risk of Alzheimer's dementia.

### Brief Description of Drawings

[Fig. 1] Fig. 1 shows graphs of the relative abundances of 4 bacterial species, of 7 bacterial species belonging to the first group, in patients with Alzheimer's dementia (AD), healthy volunteers (HV) and the risk group (RG) for AD dementia.
[Fig. 2] Fig. 2 shows graphs of the relative abundances of 4 bacterial species, of the 7 bacterial species belonging to the first group, in patients with Alzheimer's dementia (AD), healthy volunteers (HV) and the risk group (RG) for AD dementia.
[Fig. 3] Fig. 3 shows graphs of the relative abundances of 4 bacterial species, of 17 bacterial species belonging to the second group, in patients with Alzheimer's dementia (AD), healthy volunteers (HV) and the risk group (RG) for AD dementia.
[Fig. 4] Fig. 4 shows graphs of the relative abundances of 4 bacterial species, of the 17 bacterial species belonging to the second group, in patients with Alzheimer's dementia (AD), healthy volunteers (HV) and the risk group (RG) for AD dementia.
[Fig. 5] Fig. 5 shows graphs of the relative abundances of 4 bacterial species, of the 17 bacterial species belonging to the second group, in patients with Alzheimer's dementia (AD), healthy volunteers (HV) and the risk group (RG) for AD dementia.
[Fig. 6] Fig. 6 shows graphs of the relative abundances of 5 bacterial species, of the 17 bacterial species belonging to the second group, in patients with Alzheimer's dementia (AD), healthy volunteers (HV) and the risk group (RG) for AD dementia.

### Description of Embodiments

### [Description of Embodiments of The Present Disclosure]

First, embodiments of the present disclosure will be listed and described.

The method of assessing the risk of Alzheimer's dementia according to the present disclosure includes the steps of:
extracting DNA contained in a test sample from the test sample;
identifying bacterial species contained in the test sample based on the sequence data of the extracted DNA;
outputting the abundances of bacterial species belonging to a first group and bacterial species belonging to a second group, or the relative abundance ratio of the bacterial species belonging to the first group and the bacterial species belonging to the second group, of the bacteria contained in the test sample; and
assessing the abundances or the relative abundance ratio.
The test sample is a fecal sample. The first group includes bacterial species associated with Alzheimer's dementia. The second group includes bacterial species associated with the inhibition of Alzheimer's dementia.

Alzheimer's dementia (AD), which accounts for the majority of dementia, is a progressive neurological disorder caused by amyloid beta (Aβ) accumulation in the brain and tau protein phosphorylation. While Alzheimer's dementia progresses from a healthy state through a mild cognitive impairment (MCI) to the onset thereof, a recent study reports the finding that Aβ accumulation in the brain already starts in a healthy state, and it takes 20 to 30 years from the start of Aβ accumulation to the onset of AD dementia. As described above, a group of individuals in whom Aβ accumulation is observed but who still have normal cognitive function is referred to as "risk group for AD dementia". If it is possible to detect the risk group for AD dementia in an early stage and to prevent the progression of Aβ accumulation, it is thought that the transition MCI and AD can be delayed or prevented. Therefore, it is desired to be able to detect Aβ accumulation in an early stage. On the other hand, there is a report that gut bacteria belonging to the order *Clostridiales* control Aβ accumulation in the brain, in mice. There is also a report that a group of bacteria, among gut bacteria, which are associated with Aβ production or Aβ degradation. However, there has been no finding on Aβ production or Aβ degradation at the level of a specific species in gut bacteria in humans.

Under such circumstances, a novel method of assessing the risk of Alzheimer's dementia was examined. The inventor has discovered that there are differences in the relative abundances of specific bacterial species among the gut microbiota of healthy volunteers, the risk group for AD dementia, and patients with AD dementia. Further, the inventor has discovered that it is possible to screen the risk group for AD dementia, by: calculating the abundances or the relative abundances of the first group that includes bacterial species associated with Alzheimer's dementia and the second group that includes bacterial species associated with the inhibition of Alzheimer's dementia, of the gut microbiota of each subject; and assessing the abundances or the relative abundances, thereby arriving at the method according to the present disclosure.

The present inventor conceived the idea that it is possible to assess the risk of Alzheimer's dementia, by collectively analyzing the gut microbiota contained in a sample obtained from a subject; and by classifying the bacteria contained in the gut microbiota into a group (first group) that includes bacterial species associated with Aβ production and a group (second group) that includes bacterial species associated with the inhibition of Aβ production, calculating the amounts or the relative abundances of the bacterial species of these groups, and using the respective amounts of the first group and the second group or the relative abundance ratio of the first group and the second group as indices. As a result of further examination, it has been found that this assessment method enables not only to distinguish between AD patients and healthy volunteers but also to distinguish the risk group for AD dementia, and is useful as a method of assessing the risk of AD dementia, thereby completing the present invention. According to the present disclosure, a novel method of assessing the risk of AD dementia is provided.

In the method of assessing the risk of Alzheimer's dementia, the bacterial species associated with Alzheimer's dementia may be bacterial species associated with amyloid beta production, and the bacterial species associated with the inhibition of Alzheimer's dementia may be bacterial species associated with the inhibition of amyloid beta production.

In the method of assessing the risk of Alzheimer's dementia, the first group may be a group including *Blautia obeum, Ruminococcus torques* and *Subdoligranulum variabile,* or a group including *Subdoligranulum variabile* and *Ruthenibacterium lactatiformans*. The second group may be a group including *Phocaeicola vulgatus* 1, *Blautia wexlerae* and *Clostridium butyricum*.

The first group may further include one or more selected from the group consisting of bacteria belonging to the genus *Romboutsia*, bacteria belonging to the family *Lachnospiraceae*, bacteria belonging to the genus *Terrisporobacter*, and bacteria belonging to the genus *Bacteroides*. The second group may further include one or more selected from the group consisting of bacteria belonging to the genus *Agathobacter*, bacteria belonging to the genus *Bifidobacterium*, bacteria belonging to the genus *Agathobaculum*, bacteria belonging to the genus *Faecalibacterium*, bacteria belonging to the genus *Roseburia*, bacteria belonging to the genus *Blautia*, bacteria belonging to the genus *Ruminococcus*, bacteria belonging to the genus *Phocaeicola*, bacteria belonging to the genus *Lactobacillus*, and bacteria belonging to the genus *Bacteroides*.

In the method of assessing the risk of Alzheimer's dementia, the test sample may be each of a first test sample obtained at a first time point and a second test sample obtained at a second time point different from the first time point, both of which are obtained from the same subject, as the test sample. The method of assessing the risk of Alzheimer's dementia may include the step of comparing:
the abundances of the bacterial species belonging to the first group and the bacterial species belonging to the second group, or the relative abundance ratio of the bacterial species belonging to the first group and the bacterial species belonging to the second group, in the first test sample, and
the abundances of the bacterial species belonging to the first group and the bacterial species belonging to the second group, or the relative abundance ratio of the bacterial species belonging to the first group and the bacterial species belonging to the second group, in the second test sample.

In the method of assessing the risk of Alzheimer's dementia, the step of identifying bacteria contained in the test sample based on the sequence data of the extracted DNA, may be performed using 16S rRNA gene sequencing.

The apparatus for assessing the risk of Alzheimer's dementia according to the present disclosure includes:
a microbiome data acquisition unit that extracts DNA contained in a test sample from the test sample, and identifies bacterial species contained in the test sample based on the sequence data of the extracted DNA;
a data derivation unit that outputs the abundances of bacterial species belonging to a first group and bacterial species belonging to a second group, or the relative abundance ratio of the bacterial species belonging to the first group and the bacterial species belonging to the second group, of the bacteria contained in the test sample, based on the microbiome data of the test sample, acquired by the microbiome data acquisition unit; and
an assessment unit that assesses the risk of Alzheimer's dementia based on the result derived by the data derivation unit.
The test sample is a fecal sample. The first group includes bacterial species associated with Alzheimer's dementia. The second group includes bacterial species associated with the inhibition of Alzheimer's dementia.

### [Details of Embodiments of the Present Disclosure]

The assessment method and the assessment apparatus according to the present disclosure will be described in further detail.

### [Assessment Method]

The assessment method according to the present disclosure includes the steps of: extracting DNA contained in a test sample from the test sample; and identifying bacterial species contained in the test sample based on the sequence data of the extracted DNA.

The test sample in the assessment method according to the present disclosure is not particularly limited, as long as the test sample is obtained from a subject whose microbiota can be collected. Examples of the subject include humans, mammals other than humans (such as monkeys, cows, horses, pigs, mice, rats, guinea pigs, hamsters, dogs, cats, rabbits, sheep and goats). A preferred subject is a human. The subject characteristics are not particularly limited, and the subject may be, for example, a male or female subject aged 30 years or older, or 40 years or older. In the assessment method according to the present disclosure, the test sample is a fecal sample obtained from the subject.

The test sample may be stored until subjected to the assessment. The storage conditions are not particularly limited, and may be, for example, frozen storage, freeze drying storage and refrigerated storage conditions. The method of frozen storage may specifically be, for example, storage at an extremely low temperature using dry ice, an ultra-low temperature refrigerator, liquid nitrogen or the like.

The specific method of extracting DNA from a test sample is not particularly limited, and DNA can be extracted in accordance with a known method. Specifically, the extraction of DNA can be performed, for example, by the following procedure. A fecal sample obtained from a subject, about the size of a rice grain (about from 10 mg to 1 g), for example, is suspended in an extraction solvent, such as, for example, a Tris/HCl buffer solution. A crushing treatment is performed by adding a surfactant, a chelating agent, a digestive enzyme, glass beads and/or the like, to the extraction solvent in which the fecal sample has been suspended. The resulting solution after the crushing treatment is centrifuged to obtain a supernatant. A DNA extract can be obtained by repeating the centrifugation and the extraction as necessary. In the extraction of DNA, it is also possible to use a stool collection kit for gut microbiome analysis, which is commercially available in the market.

The microbiota contained in the test sample is analyzed, using the DNA extract obtained from the test sample as the material, thereby identifying bacterial species contained in the test sample. The microbiota contained in the test sample reflects the gut microbiota of the subject. Examples of the method of analyzing the bacteria contained in the microbiota, as well as the abundances and the relative abundances thereof include methods such as Gram staining, microscopic examination and PCR. However, the PCR method is preferred, because of its advantage that the bacteria contained in the microbiota and the relative abundances thereof can be analyzed simultaneously. In particular, it is preferred to perform a microbiome analysis using 16S rRNA, which is an amplicon sequencing analysis. In the case of performing a microbiome analysis using 16S rRNA, the genomic region to be analyzed is not particularly limited as long as the bacteria can be identified. However, a region centered around the V3-V4 region of the 16S rRNA gene is mainly used.

In the case of analyzing the bacteria contained in the microbiota and the relative abundances thereof, using 16S rRNA gene sequencing, it is preferred to use a next-generation sequencer because a plurality of samples can be analyzed simultaneously. The next-generation sequencer is not particularly limited as long as the sequencer is capable of determining the nucleotide sequences of randomly cleaved DNA fragments simultaneously, and may specifically be, for example, a MiSeq system manufactured by Illumina, Inc. or the like. The bacteria that make up the microbiota can be identified referring to the data obtained from the next-generation sequencer, and a reference genome database which is a microbial identification database. The microbial identification database is not particularly limited as long as the bacteria that make up the microbiota can be identified. The abundances or the relative abundances of specific bacterial species in the microbiota can be determined by statistically processing the results of the amplicon sequencing analysis.

The method of calculating the relative abundance of each bacterial species is not particularly limited. For example, the relative abundance may be calculated using a method in which sampling errors are corrected based on statistical methods, or may be calculated as the amount of nucleic acids derived from each bacterium with respect to the total amount of nucleic acids in the test sample. The calculation may be performed in conjunction with the analysis of the microbiota, using commercially available software.

The method according to the present disclosure includes the step of outputting the abundances of bacterial species belonging to a first group and bacterial species belonging to a second group, or the relative abundance ratio of the bacterial species belonging to the first group and the bacterial species belonging to the second group, of the bacterial species contained in the test sample.

The bacterial species belonging to the first group are bacterial species associated with Alzheimer's dementia (associated with the onset of Alzheimer's dementia), and more specifically, bacterial species associated with amyloid beta production. The bacterial species belonging to the first group and associated with amyloid beta production are a plurality of bacterial species. Specifically, the first group may be composed of three or more bacterial species, and is also preferably composed of five or more, seven or more, or ten or more bacterial species. Specifically, the first group preferably includes *Blautia obeum*, *Ruminococcus torques* and Subdoligranulum variabile. Further, the first group preferably includes *Subdoligranulum variabile* and *Ruthenibacterium lactatiformans*. It is preferred that the first group further include one or more selected from the group consisting of bacteria belonging to the genus *Romboutsia*, bacteria belonging to the family *Lachnospiraceae*, bacteria belonging to the genus *Terrisporobacter*, bacteria belonging to the genus *Bacteroides*, and bacteria belonging to the genus *Ruthenibacterium*. It is more preferred that the first group further include *Romboutsia ilealis*, *Roseburia inulinivorans*, *Terrisporobacter petrolearius*, *Bacteroides stercoris* and *Ruthenibacterium lactatiformans*. Typically, the first group may be a group consisting of eight bacterial species, namely, *Blautia obeum, Ruminococcus torques, Subdoligranulum variabile, Romboutsia ilealis, Roseburia inulinivorans, Terrisporobacter petrolearius, Bacteroides stercoris* and *Ruthenibacterium lactatiformans*. The first group can be composed of two or more, or three or more, preferably five or more, more preferably seven or more bacterial species selected from the group consisting of *Blautia obeum, Ruminococcus torques, Subdoligranulum variabile, Romboutsia ilealis, Roseburia inulinivorans, Terrisporobacter petrolearius, Bacteroides stercoris* and *Ruthenibacterium lactatiformans*. Previous studies have reported findings that bacteria belonging to the order *Clostridiales* are associated with Aβ accumulation in mice, but there has been no finding at the species level in humans. The method according to the present disclosure is characterized in that the presence of bacteria including specific bacterial species as the first group is assessed. The first group is a group that serves as an index for assessing the fact that the risk of Alzheimer's dementia is present (a negative factor for Alzheimer's dementia).

The abundance of the bacterial species belonging to the first group may be outputted as the sum of the abundances of individual bacterial species, based on taxonomic assignments at the species level identified using a microbial identification database, or may be outputted as the amount of the bacterial species belonging to the first group determined by detecting the species collectively. The relative abundance of the bacterial species belonging to the first group can be outputted as the relative abundance of the bacterial species belonging to the first group with respect to the total microbiota contained in the test sample.

The bacterial species belonging to the second group are bacterial species associated with the inhibition of Alzheimer's dementia, and more specifically, bacterial species associated with the inhibition of amyloid beta production. The bacterial species belonging to the second group are a plurality of bacterial species. Specifically, the second group may be composed of three or more bacterial species, and is also preferably composed of 5 or more, 7 or more, 10 or more, 15 or more, or 17 or more bacterial species. Specifically, the second group may include *Phocaeicola vulgatus* 1*, Blautia wexlerae and Clostridium butyricum.* It is preferred that the second group further include one or more selected from the group consisting of bacteria belonging to the genus *Agathobacter*, bacteria belonging to the genus *Bifidobacterium*, bacteria belonging to the genus *Agathobaculum*, bacteria belonging to the genus *Faecalibacterium*, bacteria belonging to the genus *Roseburia*, bacteria belonging to the genus *Blautia*, bacteria belonging to the genus *Ruminococcus*, bacteria belonging to the genus *Phocaeicola*, bacteria belonging to the genus *Lactobacillus*, and bacteria belonging to the genus *Bacteroides*. It is more preferred that the second group further include *Agathobacter rectalis, Bifidobacterium breve, Bifidobacterium longum, Agathobaculum butyriciproducens, Faecalibacterium prausnitzii, Roseburia intestinalis, Blautia hansenii*/*hominis, Blautia faecis, Ruminococcus gnavus, Roseburia hominis, Phocaeicola vulgatus* 4*, Lactobacillus acidophilus, Bacteroides uniformis* and *Bacteroides caccae*. Typically, the second group may be a group consisting of 17 bacterial species, namely, *Phocaeicola vulgatus* 1*, Blautia wexlerae, Clostridium butyricum, Agathobacter rectalis, Bifidobacterium breve, Bifidobacterium longum, Agathobaculum butyriciproducens, Faecalibacterium prausnitzii, Roseburia intestinalis, Blautia hansenii*/*hominis, Blautia faecis, Ruminococcus gnavus, Roseburia hominis, Phocaeicola vulgatus* 4*, Lactobacillus acidophilus, Bacteroides uniformis* and *Bacteroides caccae*. The second group can be composed of two or more, or three or more, preferably seven or more, more preferably ten or more bacterial species selected from the group consisting of *Phocaeicola vulgatus* 1*, Blautia wexlerae, Clostridium butyricum, Agathobacter rectalis, Bifidobacterium breve, Bifidobacterium longum, Agathobaculum butyriciproducens, Faecalibacterium prausnitzii, Roseburia intestinalis, Blautia hansenii*/*hominis, Blautia faecis, Ruminococcus gnavus, Roseburia hominis, Phocaeicola vulgatus* 4*, Lactobacillus acidophilus, Bacteroides uniformis* and *Bacteroides caccae*. The method according to the present disclosure is characterized in that the presence of bacteria including specific bacterial species as the second group is assessed. The second group is a group that serves as an index for assessing a decrease in the risk of Alzheimer's dementia (a positive factor for Alzheimer's dementia).

The abundance of the bacterial species belonging to the second group may be outputted as the sum of the abundances of individual bacterial species, based on taxonomic assignments at the species level identified using a microbial identification database, or may be outputted as the amount of the bacterial species belonging to the second group determined by detecting the species collectively. The relative abundance of the bacterial species belonging to the second group can be outputted as the relative abundance of the bacterial species belonging to the second group with respect to the total microbiota contained in the test sample.

The method according to the present disclosure includes the step of assessing the abundances or the relative abundances of the bacterial species belonging to the first group and the bacterial species belonging to the second group. Typically, the risk of AD dementia is assessed based on the ratio of the relative abundance of the bacterial species belonging to the first group and the relative abundance of the bacterial species belonging to the second group. The risk of Alzheimer's dementia is classified, for example, into three stages. The risk may be classified, for example, into three stages, namely, healthy / risk group for AD dementia/ MCI or AD dementia. The risk of Alzheimer's dementia may be susceptibility to Alzheimer's dementia, or the probability of progression to MCI. Further, the risk may also be health status (physical characteristics such as body weight and body height, diet, exercise, sleep, etc.) or the probability of changes (deterioration) in cognitive function, mental function and/or motor function, that serves as a preliminary index of the onset of Alzheimer's dementia and/or MCI.

Specifically, for example, when the abundance of the bacterial species belonging to the first group is extremely low and the abundance of the bacterial species belonging to the second group is high in a subject, the subject is assessed as healthy. When the abundance of the bacterial species belonging to the first group shows an increasing trend and the abundance of the bacterial species belonging to the second group shows a decreasing trend in a subject, the subject is assessed as falling into the risk group for Alzheimer's dementia. When the abundance of the bacterial species belonging to the first group is high and the abundance of the bacterial species belonging to the second group is extremely low in a subject, the subject is assessed as having MCI or Alzheimer's dementia. Objective and/or empirical criteria can be used as the assessment criteria. Further, the assessment criteria can be established using artificial intelligence.

In the method according to the present disclosure, it is possible to obtain test samples at a plurality of time points from the same subject, and to assess changes in the abundance profiles of the bacterial species belonging to the first group and the bacterial species belonging to the second group in the gut microbiota. In other words, a first test sample obtained at a first time point, and a second test sample obtained at a second time point different from the first time point are used as test samples. The interval between the first time point and the second time point may be any time interval, such as, for example, one month or more, six months or more, one year or more, or three years or more. The plurality of time points may be two time points, or three or more time points. For example, the plurality of time points may be two or more time points set at a time interval of every six months, every year or the like. The risk of Alzheimer's dementia can be assessed more accurately by obtaining test samples at a plurality of time points.

The respective abundances and the relative abundances of the bacterial species belonging to the first group and the bacterial species belonging to the second group, of the plurality of samples at the plurality of time points, may be analyzed respectively at the time points of obtaining the samples, or may be analyzed simultaneously after obtaining the samples at the plurality of time points and storing the samples.

In the method of assessing the risk of Alzheimer's dementia according to the present disclosure, the assessment method based on the abundances or the relative abundances of the bacterial species of the first group and the second group described above may be combined with another assessment index. The assessment index to be combined with the assessment method according to the present disclosure may be, for example, one or a plurality of indices selected from the group consisting of gender, age, body height, body weight, blood pressure, lifestyle habits, visual acuity, hearing and the like. The assessment index may be a test result of a cognitive function test, a mental function test, a motor function test, etc. Further, the assessment index may be a diagnosis result of MRI imaging, etc. The assessment index may also be, for example, past surgical history, past medical history, medication history, the presence or absence of any disease currently being treated, etc.

### [Assessment Apparatus]

The assessment apparatus according to the present disclosure is an apparatus suitable for performing the assessment method described above. The assessment apparatus according to the present disclosure includes a microbiome data acquisition unit that extracts DNA contained in a test sample from the test sample, and estimates bacterial species contained in the test sample based on the sequence data of the extracted DNA. The data acquisition unit can include a pre-treatment device such as an automated DNA extractor, a PCR device, a device such as a sequencer, etc. Further, the data acquisition unit can include a control section for executing processing by these devices. Automated processing executed by a command from the control section may be combined with manual operation by a human operator.

The assessment apparatus according to the present disclosure includes a data derivation unit that outputs the respective abundances of bacterial species belonging to a first group and bacterial species belonging to a second group, or the relative abundance ratio of the bacterial species belonging to the first group and the bacterial species belonging to the second group, of the bacteria contained in the test sample, based on the microbiome data of the test sample, acquired by the microbiome data acquisition unit. The data derivation unit includes software, and may include a control section for allowing the software to execute a statistical processing.

The assessment apparatus according to the present disclosure includes an assessment unit that assesses the risk of Alzheimer's dementia based on the result derived by the data derivation unit. The assessment unit may perform the assessment based on the data provided by the data derivation unit, or may perform the assessment by combining the data provided by the data derivation unit and information obtained from a database in which gut microbiota profiles are associated with the risk of dementia.

### [Assessment Method and Use of Assessment Results]

The information of the risk of Alzheimer's dementia obtained by the assessment method according to the present disclosure can be used as information for providing the risk of Alzheimer's dementia to the subjects.

Further, the information of the risk of Alzheimer's dementia obtained by the assessment method according to the present disclosure can be used as information for assessing the nootropic effect of a food or supplement. In the case of using the thus obtained information as information for assessing the nootropic effect of a food or supplement, for example, a plurality of subjects (such as 100, 300 or 500 subjects) are asked to ingest the food or supplement to be assessed, for a certain number of times (or for a certain period of time, at a certain interval, or in a certain amount). The nootropic effect of the food or supplement can be assessed by analyzing changes in the relative abundances of the bacterial species belonging to the first group and the bacterial species belonging to the second group before and after the ingestion. The nootropic effect can also be assessed by analyzing an increase or a decrease in the amount of bacterial species belonging to the first group before and after the ingestion, and an increase or a decrease in the amount of bacterial species belonging to the second group before and after the ingestion. The assessment method according to the present disclosure can be used not only for assessing the nootropic effect of a food or supplement, but also for the development of a novel food or supplement that has a nootropic effect.

### [Examples]

Examples of the assessment method according to the present disclosure will be shown below.

### [Preparation and Analysis of Test Samples]

A fecal sample was obtained from each of healthy volunteers (HV), the risk group (RG) for AD dementia and patients with Alzheimer's dementia (AD) as subjects. Individuals taking medication (PPI, PCAB, drugs for treating DM, antibiotics and antithrombotic drugs) which have an impact on the gut microbiota, and individuals with unbalanced diets were excluded.

All of the subjects underwent brain imaging, blood tests for thyroid function and vitamin levels, and MMSE which is a neuropsychological test. HV and AD were selected based on a diagnosis by a dementia specialist or a neurologist. As the risk group (RG) for AD dementia, individuals aged 40 years or older as candidates underwent cognitive function tests, etc., and those who were determined to belong to the risk group for AD dementia in advance, were selected.

Eight samples were obtained from the healthy volunteers (HV), five samples were obtained from the risk group (RG) for AD dementia, and ten samples were obtained from the patients with Alzheimer's dementia (AD).

### 1. Amplicon Sequencing Analysis

(1) DNA was extracted from each fecal sample. For the extraction of DNA, pre-treatment was performed in accordance with the method of Takahashi et al. (Reference 1). The resulting crude DNA extract was purified using an automated DNA extractor, (GENE PREP STAR PI-480, manufactured by Kurabo Industries Ltd.).
(2) PCR amplification was performed in accordance with the method of Takahashi et al. (Reference 1), using a primer set (341f-R806) (References 2 and 3) for amplifying the V3-V4 region of the 16S rRNA gene of bacteria. At this time, a sample-specific index sequence (Reference 4) was inserted into each primer.
(3) Paired-end sequencing was performed for sequence determination, using a Miseq system (manufactured by Illumina, Inc.) and a MiSeq Reagent Kit v 3 (600 cycles) (manufactured by Illumina, Inc.) with 2 × 301 bp cycles.

### 2. Analysis of Amplicon Sequencing Data

(1) After the paired-end sequencing, the resulting reads were trimmed to remove primer sequences using the Cutadapt software, version 1.18 (Reference 5) with the default settings.
(2) The paired-end reads were merged using the fastq-join software (Reference 6) with the default settings.
(3) Reads in which 99% or more of the bases have a quality value (QV) of 20 or more were selected using the FASTX-Toolkit software, version 0.0.14 (Reference 7), and chimeric sequences were removed using the usearch61 software (References 8 and 9).
(4) After removing the chimeric sequences, the taxonomic assignment of nucleotide sequences was performed using the Metagenoem@KIN software, version 2.2.1, and using RDP version 2.13 (Reference 10) and a microbial identification database, NGS-DB-BA 16.04 (Reference 11) (manufactured by TechnoSuruga Laboratory Co., Ltd.), as databases. The taxonomic assignment was performed with a confidence of 0.8 or more in the case of using RDP and with a homology of 97% or more in the case of using the microbial identification database.

### 3. Data Analysis by Statistical Analysis Software R

(1) The composition ratios of the bacterial species associated with amyloid beta production (Table 1) and the bacterial species associated with the inhibition of amyloid beta production (Table 2) were extracted, based on taxonomic assignments at the species level identified using a microbial identification database.
(2) Using the extracted composition ratios of the respective bacterial species, box plots were drawn using the statistical analysis software R, version 3.1.01 (Reference 12) based on the grouping information.
(3) To determine the presence or absence of significant differences in the composition ratios between the groups, Mann-Whitney U tests (Reference 15) were performed for each bacterial species, using the vegan package, version 2.5.7 (References 13 and 14). Statistical tests were performed to calculate statistics and p-values. False discovery rate (FDR) correction of p-values was performed using the Bonferroni method (Reference 16).

Heatmap clustering and alpha and beta diversity analyses were also performed based on the nucleotide sequence data obtained from each fecal sample, and the results confirmed that the composition of the gut microbiota varies between the HV group, the RG group and the AD group.

**[Table 1]**

| Bacterial species associated with Aβ production | |
|---|---|
| | *Blautia obeum* |
| | *Romboutsia ilealis* |
| | *Roseburia inulinivorans* |
| **First Group** | *Ruminococcus torques* |
| | *Subdoligranulum variabile* |
| | *Terrisporobacter petrolearius* |
| | *Bacteroides stercoris* |
| | *Ruthenibacterium lactatiformans* |

**[Table 2]**

| Bacterial species associated with the inhibition of Aβ production | |
|---|---|
| | *Agathobaculum butyriciproducens* |
| | *Faecalibacterium prausnitzii* |
| | *Roseburia hominis* |
| | *Clostridium butyricum* |
| | *Agathobacter rectalis* |
| | *Bifidobacterium breve* |
| | *Bifidobacterium longum* |
| **Second group** | *Lactobacillus acidophilus* |
| | *Blautia wexlerae* |
| | *Roseburia intestinalis* |
| | *Blautia hansenii* / *hominis* |
| | *Blautia faecis* |
| | *Phocaeicola vulgatus* 1 |
| | *Phocaeicola vulgatus* 4 |
| | *Bacteroides uniformis* |
| | *Bacteroides caccae* |
| | *Ruminococcus gnavus* |

### [Results]

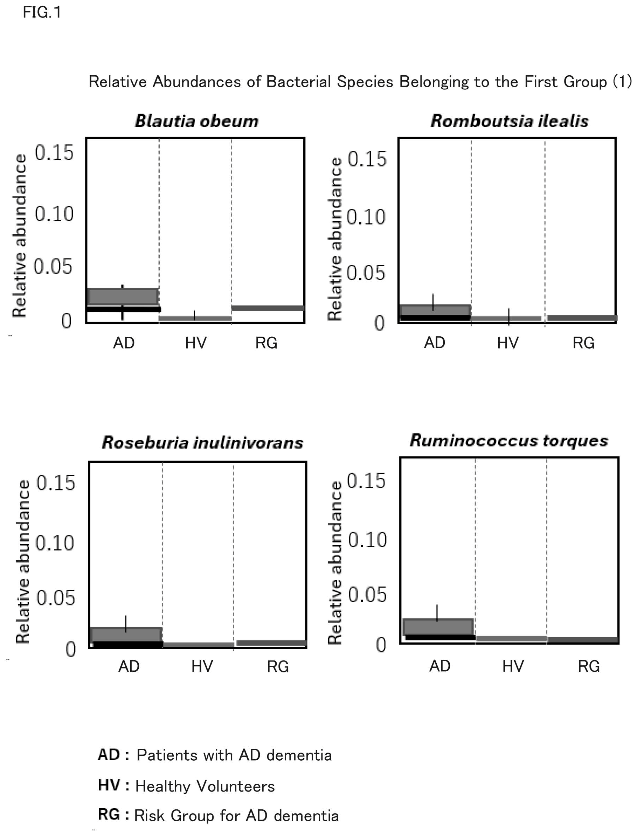

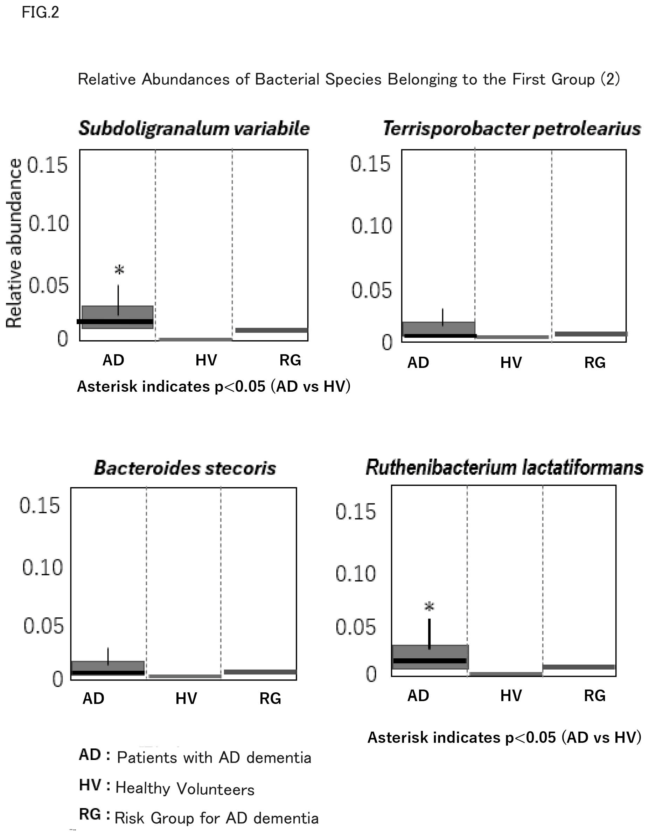

Fig. 1 and Fig. 2 show graphs of the relative abundances of the respective eight bacterial species belonging to the first group, in patients with Alzheimer's dementia (AD), healthy volunteers (HV) and the risk group (RG) for AD dementia. As shown in Fig. 1 and Fig. 2, the relative abundance of each of the eight bacterial species was higher in the AD group than in the HV group and the RG group. Further, the relative abundance tended to differ between the AD group, the HV group and the RG group, in each bacterial species. In particular, there was a clear tendency that the relative abundances of three bacterial species, namely, *Blautia obeum, Ruminococcus torques* and *Subdoligranulum variabile*, are higher in the AD group. This confirmed the fact that the group of bacterial species including the three bacterial species, *Blautia obeum, Ruminococcus torques* and *Subdoligranulum variabile* can be used as a parameter for assessing the risk of developing Alzheimer's dementia, as the group associated with Alzheimer's dementia. Further, a significant difference of p < 0.05 was observed between the AD group and the HV group, in two bacterial species, *Subdoligranulum variabile* and *Ruthenibacterium lactatiformans*. It has been confirmed that the group of bacterial species including the eight bacterial species shown in Table 1 can be used as a parameter for assessing the risk of developing Alzheimer's dementia, as the group associated with Alzheimer's dementia.

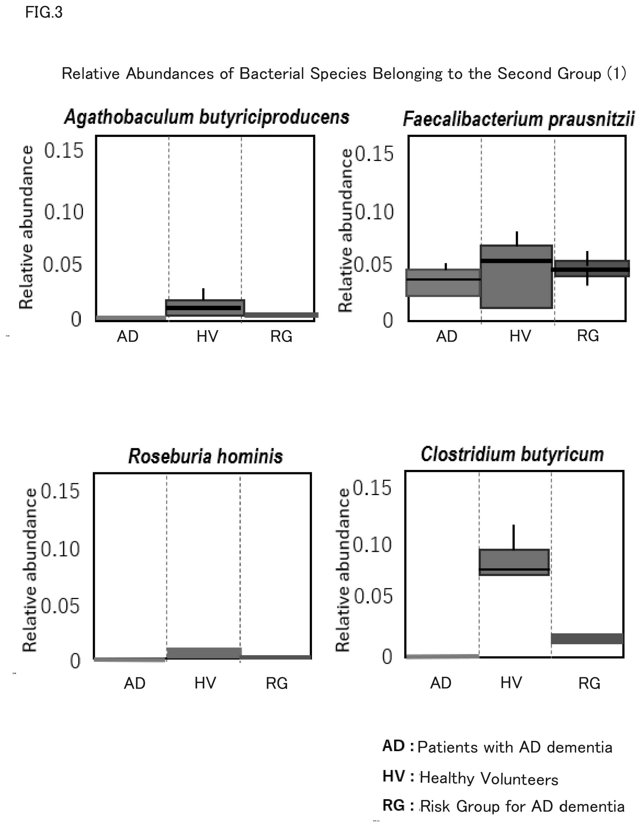

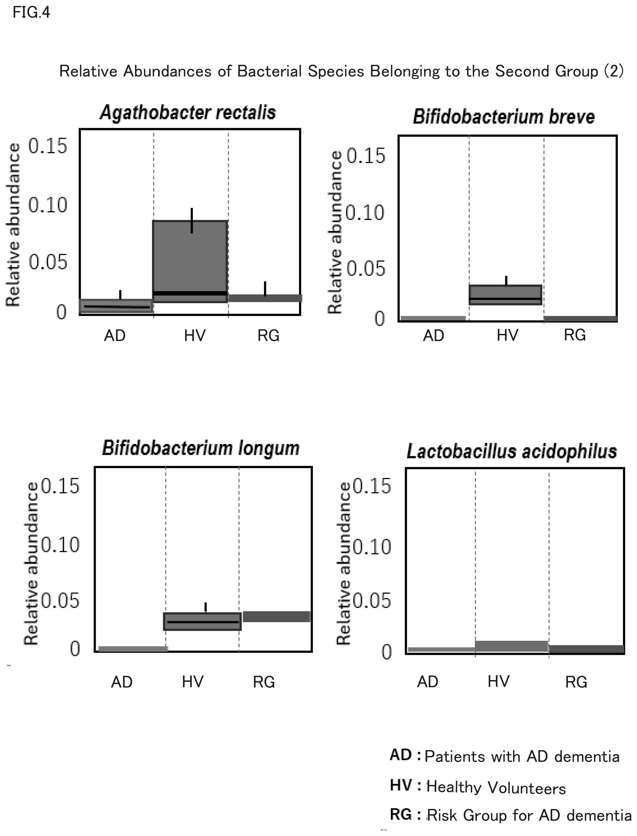

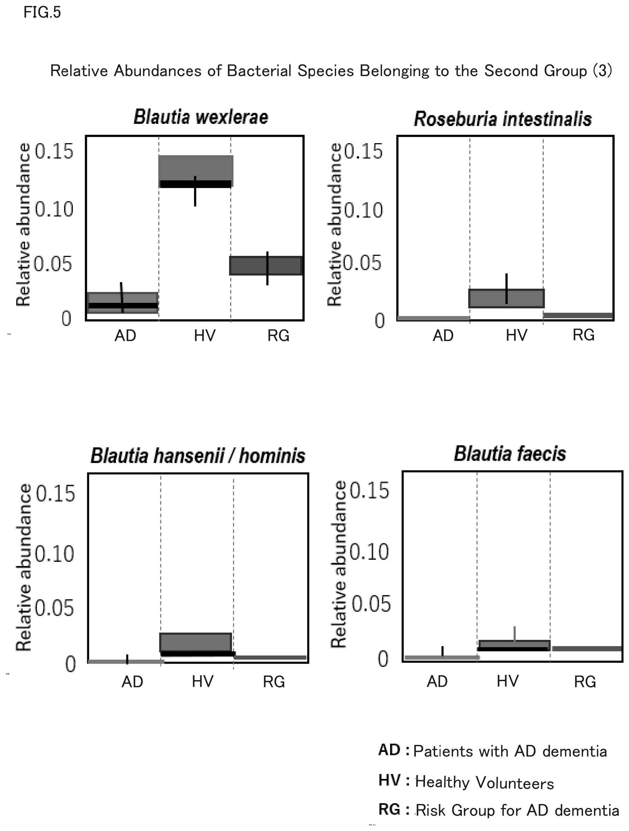

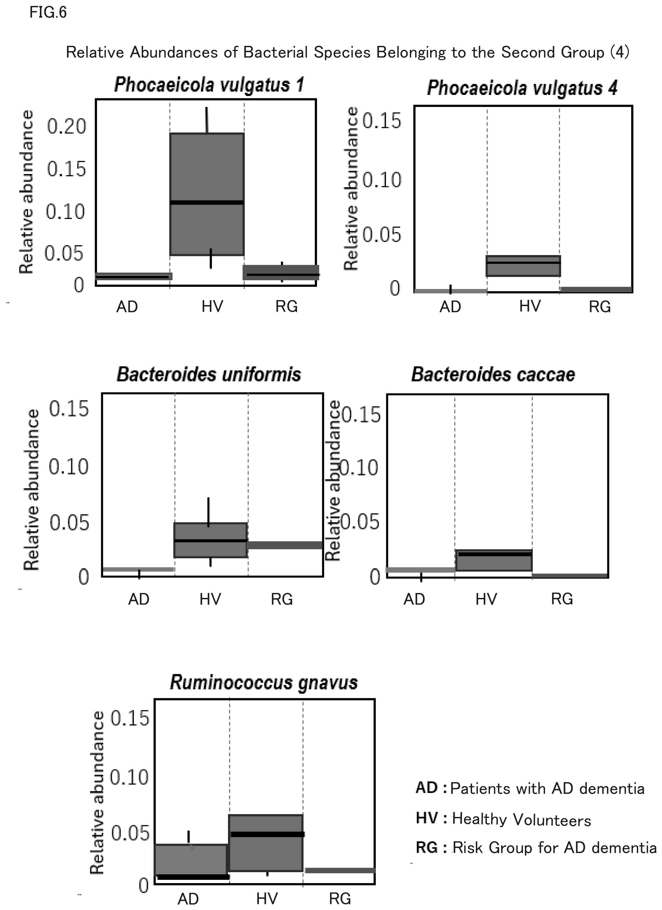

Fig. 3 to Fig. 6 show graphs of the relative abundances of the respective 17 bacterial species belonging to the second group, in patients with Alzheimer's dementia (AD), healthy volunteers (HV) and the risk group (RG) for AD dementia. As shown in Fig. 3 to Fig. 6, the relative abundance was higher in the HV group than in the RG group and the AD group, in all of the 17 bacterial species. Further, the relative abundance tended to differ between the AD group, the HV group and the RG group, in each bacterial species. In particular, there was a clear tendency that the relative abundances of three bacterial species, namely, *Phocaeicola vulgatus* 1*, Blautia wexlerae* and *Clostridium butyricum*, are higher in the HV group. This confirmed the fact that the group of bacterial species including the three bacterial species, *Phocaeicola vulgatus* 1, *Blautia wexlerae* and *Clostridium butyricum*, can be used as a parameter for assessing the risk of developing Alzheimer's dementia, as the group associated with the inhibition of Alzheimer's dementia. Further, it has been confirmed that the group of bacterial species including the 17 bacterial species shown in Table 2 can be used as a parameter for assessing the risk of developing Alzheimer's dementia, as the group associated with the inhibition of Alzheimer's dementia.

As shown in Figs. 1 to 6, the relative abundances of the bacteria of the first group that includes bacterial species associated with Alzheimer's dementia, and the relative abundances of the bacteria of the second group that includes bacterial species associated with the inhibition of Alzheimer's dementia, differ among healthy volunteers (HV), the risk group (RG) for AD dementia and patients with Alzheimer's dementia (AD). Particularly in the risk group (RG) for AD dementia, it has been confirmed that the balance of the gut bacterial species is different from that of the healthy volunteers (HV) even though the cognitive function is normal, and approaching the balance of the gut bacterial species of the patients with AD dementia, and it is thought that the subjects belonging to the risk group for AD dementia can be screened. Further, it has been confirmed that healthy volunteers, the risk group for AD dementia and patients with Alzheimer's dementia can be screened based on the balance between the relative abundance of the bacterial species of the first group and the relative abundance of the bacterial species of the second group.

### (References)

1. Takahashi S, Tomita J, Nishioka K, Hisada T, Nishijima M. Development of a prokaryotic universal primer for simultaneous analysis of Bacteria and Archaea using next-generation sequencing. PLoS One 2014;9: e105592.
2. uyzer G, de Waal, EC. Uitterlinden, AG. Profiling of complex microbial populations by denaturing gradient gel electrophoresis analysis of polymerase chain reaction-amplified genes coding for 16S rRNA. Appl Environ Microbiol 1993; 59:695-700.
3. Caporaso JG, Lauber CL, Walters WA, Berg-Lyons D, Lozupone CA et al. Global patterns of 16S rRNA diversity at a depth of millions of sequences per sample. Proc Natl Acad Sci USA 2011; 108:4516-4522.
4. Hisada T, Endoh K, Kuriki K. Inter-and intra-individual variations in seasonal and daily stabilities of the human gut microbiota in Japanese. Arch Microbiol 2015; 197:919-934.
5. Martin M. Cutadapt removes adapter sequences from high-throughput sequencing reads. EMBnet. journal 2011; 17:10-12.
6. Aronesty E. Comparison of sequencing utility programs. Open Bioinforma J 2013; 7:1-8.
7. Gordon A, Hannon GJ. FASTX-Toolkit FASTQ/A short-reads preprocessing tools [software]. Available from: http://hannonlab.cshl.edu/fastx_toolkit/index.html
8. Caporaso JG, Kuczynski J, Stombaugh J, Bittinger K, Bushman FD et al. QIIME allows analysis of high-throughput community sequencing data. Nat Methods 2010; 7:335336.
9. Edgar RC, Haas BJ, Clemente JC, Quince C, Knight R. UCHIME improves sensitivity and speed of chimera detection. Bioinformatics 2011; 27:2194-2200.
10. Wang Q, Garrity GM, Tiedje JM, Cole JR. Naive bayesian classifier for rapid assignment of rRNA sequences into the new bacterial taxonomy. Appl Environ Microbiol 2007; 73:5261-5267.
11. Kasai C, Sugimoto K, Moritani I, Tanaka J, Oya Y et al. Comparison of the gut microbiota composition between obese and non-obese individuals in a Japanese population, as analyzed by terminal restriction fragment length polymorphism and next-generation sequencing. BMC Gastroenterol 2015; 15:100.
12. R Core Team. R: A language and environment for statistical computing. R Foundation for Statistical Computing, Vienna 2014. URL https://www.R-project.org/.
13. Dixon P. VEGAN, a package of R functions for community ecology. J Veg Sci 2003; 14:927-930
14. Oksanen J, Blanchet FG, Friendly M, Kindt R, Legendre P et al. vegan: Community Ecology Package. https://CRAN.R-project.org/package=vegan.
15. McKnight PE, Najab J. Mann-Whitney U Test. The Corsini encyclopedia of psychology 2010;1-1.
16. Abdi H. Bonferroni and Sisak corrections for multiple comparisons. In: Salkind NJ editor. Encyclopedia of measurement and statistics. Thousand Oaks: SAGE Publications; 2007. pp. 103-107.

The embodiments disclosed herein are to be considered in all respects as illustrative and not restrictive. The scope of the present invention is defined not by the above description but by the claims, and is intended to include all modifications within the meaning and scope equivalent to the claims.

## Claims

1. A method of assessing risk of Alzheimer's dementia, the method comprising the steps of:
extracting DNA contained in a test sample from the test sample;
identifying bacterial species contained in the test sample based on sequence data of the extracted DNA;
outputting abundances of bacterial species belonging to a first group and bacterial species belonging to a second group, or relative abundance ratio of the bacterial species belonging to the first group and the bacterial species belonging to the second group, of the bacterial species contained in the test sample; and
assessing the abundances or the relative abundance ratio;
wherein the test sample is a fecal sample;
wherein the first group comprises bacterial species associated with Alzheimer's dementia; and
wherein the second group comprises bacterial species associated with inhibition of Alzheimer's dementia.

2. The method of assessing risk of Alzheimer's dementia according to claim 1, wherein the first group is a group comprising *Blautia obeum*, *Ruminococcus torques* and *Subdoligranulum variabile*, or a group comprising *Subdoligranulum variabile* and *Ruthenibacterium lactatiformans*.

3. The method of assessing risk of Alzheimer's dementia according to claim 1 or claim 2, wherein the second group is a group comprising *Phocaeicola vulgatus* 1*, Blautia wexlerae* and *Clostridium butyricum*.

4. The method of assessing risk of Alzheimer's dementia according to claim 3,
wherein the first group further comprises one or more selected from the group consisting of bacteria belonging to the genus *Romboutsia,* bacteria belonging to the family *Lachnospiraceae*, bacteria belonging to the genus *Terrisporobacter*, and bacteria belonging to the genus *Bacteroides*; and
the second group further comprises one or more selected from the group consisting of bacteria belonging to the genus *Agathobacter*, bacteria belonging to the genus *Bifidobacterium*, bacteria belonging to the genus *Agathobaculum*, bacteria belonging to the genus *Faecalibacterium*, bacteria belonging to the genus *Roseburia*, bacteria belonging to the genus *Blautia*, bacteria belonging to the genus *Ruminococcus*, bacteria belonging to the genus *Phocaeicola*, bacteria belonging to the genus *Lactobacillus*, and bacteria belonging to the genus *Bacteroides*.

5. The method of assessing risk of Alzheimer's dementia according to claim 1 or claim 2,
wherein each of a first test sample obtained at a first time point and a second test sample obtained at a second time point different from the first time point, both of which are obtained from the same subject, is used as the test sample; and
wherein the method comprises the step of comparing:
the abundances of the bacterial species belonging to the first group and the bacterial species belonging to the second group, or the relative abundance ratio of the bacterial species belonging to the first group and the bacterial species belonging to the second group, in the first test sample, and
the abundances of the bacterial species belonging to the first group and the bacterial species belonging to the second group, or the relative abundance ratio of the bacterial species belonging to the first group and the bacterial species belonging to the second group, in the second test sample.

6. The method of assessing risk of Alzheimer's dementia according to claim 1 or claim 2, wherein the step of identifying bacteria contained in the test sample based on the sequence data of the extracted DNA, is performed using 16S rRNA gene sequencing.

7. An apparatus for assessing risk of Alzheimer's dementia, the apparatus comprising:
a microbiome data acquisition unit that extracts DNA contained in a test sample from the test sample, and identifies bacterial species contained in the test sample based on sequence data of the extracted DNA;
a data derivation unit that outputs abundances of bacterial species belonging to a first group and bacterial species belonging to a second group, or relative abundance ratio of the bacterial species belonging to the first group and the bacterial species belonging to the second group, of the bacterial species contained in the test sample, based on the microbiome data of the test sample, acquired by the microbiome data acquisition unit; and
an assessment unit that assesses the risk of Alzheimer's dementia based on result derived by the data derivation unit;
wherein the test sample is a fecal sample;
wherein the first group comprises bacterial species associated with Alzheimer's dementia; and
wherein the second group comprises bacterial species associated with inhibition of Alzheimer's dementia.
